# EUROPEAN PATENT APPLICATION

(11) **EP 0 613 007 A2**
(43) Date of publication of application: **31.08.1994**
(21) Application number: 94301170.0
(22) Date of filing: 18.02.1994
(51) Int. Cl.: G01N 33/68, C07K 15/28

(54) **Pharmaceutical screens and antibodies**

(30) Priority: 22.02.1993 US 21609
(71) Applicant: ELI LILLY AND COMPANY, Indianapolis, Indiana 46285 (US); ATHENA NEUROSCIENCES, INC., South San Francisco, CA 94080 (US)
(72) Inventor: Becker, Gerald Wayne, Fishers, Indiana 46038 (US); Brems, David Nettleship, Indianapolis, Indiana 46256 (US); Chaney, Michael Owen, Carmel, Indiana 46032 (US); May, Patrick Cornelious, Carmel, Indiana 46032 (US); Rydel, Russel Eugene, Belmont, California 94002 (US); Simmons, Linda Karen, Indianapolis, Indiana 46202 (US); Tomaselli, Kevin James, San Francisco, California 94133 (US)
(74) Representative: Tapping, Kenneth George

(57) **Abstract**

This invention describes a series of assays useful in evaluating the efficacy of agents which inhibit the neurotoxic effects of β-amyloid peptide. These assays employ β-amyloid peptide which is in a predominantly β-sheet conformation. This invention also encompasses antibodies having a specificity for β-amyloid peptide which is predominantly in a β-sheet conformation as well as pharmaceutic formulations containing these antibodies. These antibodies show poor reactivity with β-amyloid peptide which has a great deal of random coil or α-helix secondary structure.

## Description

Alzheimer's disease is a degenerative disorder of the human brain. Clinically, it appears as a progressive dementia. Its histopathology is characterized by degeneration of neurons, gliosis, and the abnormal deposition of proteins in the brain. Proteinaceous deposits (called "amyloid") appear as neurofibrillary tangles, amyloid plaque cores, and amyloid of the congophilic angiopathy. [For reviews, see, Alzheimer's Disease, (B. Reisberg, ed., The Free Press 1983).]

While there is no general agreement as to the chemical nature of neurofibrillary tangles, the major constituent of both the amyloid plaque cores and the amyloid of the congophilic angiopathy has been shown to be a 4500 Dalton protein originally termed β-protein or amyloid A4. Throughout this document this protein is referred to as β-amyloid peptide or protein.

β-amyloid peptide is proteolytically derived from a transmembrane protein, the amyloid precursor protein. Different splice forms of the amyloid precursor protein are encoded by a widely expressed gene. see. e.g., K. Beyreuther and B. Müller-Hill, Annual Reviews in Biochemistry, 58:287-307 (1989). β-amyloid peptide consists, in its longest forms, of 42 or 43 amino acid residues. J. Kang, et al., Nature (London), 325:733-736 (1987). These peptides, however, vary as to their amino-termini. C. Hilbich, et al., Journal of Molecular Biology, 218:149-163 (1991).

Because senile plaques are invariably surrounded by dystrophic neurites, it was proposed early that β-amyloid peptide is involved in the loss of neuronal cells that occurs in Alzheimer's disease. B. Yankner and co-workers were the first to demonstrate that synthetic β-amyloid peptide could be neurotoxic in vitro and in vivo. B.A. Yankner, et al., Science, 245:417 (1989); See, also, N.W. Kowall, et al., Proceedings of the National Academy of Sciences, U.S.A., 88:7247 (1991). Other research groups, however, were unable to consistently demonstrate direct toxicity with β-amyloid peptide. See, e.g., Neurobiology of Aging, 13:535 (K. Kosik and P. Coleman, eds. 1992). Even groups receiving β-amyloid peptide from a common source demonstrate conflicting results. D. Price, et al., Neurobiology of Aging, 13:623-625 (1991) (and the references cited therein).

Because of the debilitating effects of Alzheimer's disease there continues to exist a need for effective treatments. This invention provides assay systems which are useful to evaluate the efficacy of potential agents to treat this disease.

This invention describes a series of assays useful in evaluating the efficacy of agents which inhibit the neurotoxic effects of β-amyloid peptide. These assays employ β-amyloid peptide which is in a predominantly β-sheet conformation.

In another embodiment, this invention describes antibodies having a specificity for β-amyloid peptide which is predominantly in a β-sheet conformation. These antibodies show poor reactivity with β-amyloid peptide which has a high degree of random coil or α-helix secondary structure.

This invention also encompasses pharmaceutical formulations comprising an antibody having a specificity for β-amyloid peptide which is predominantly in a β-sheet conformation in combination with a parenterally-administrable medium.

The terms and abbreviations used in the instant examples have their normal meanings unless otherwise designated. For example "°C" refers to degrees Celsius; "N" refers to normal or normality; "mmole" refers to millimole or millimoles; "g" refers to gram or grams; "ml" means milliliter or milliliters; "M" refers to molar or molarity; and "CD" refers to circular dichroism spectrometry.

This invention depicts assays employing β-amyloid peptide in which the secondary structure of the peptide is predominantly β-sheet. The amount of β-sheet structure present in the β-amyloid peptides employed in this specification differs.

A preferred embodiment of this invention employs β-amyloid peptide which has adopted at least 65% of its potential β-sheet conformation. The most preferred embodiment of this invention employs β-amyloid peptide which has adopted at least 80% of its potential β-sheet conformation. The amount of β-sheet conformation present in a peptide lot can be readily determined by examining the circular dichroism spectrum of the peptide lot.

While this peptide is referred to as β-amyloid peptide throughout this document, in the body of literature concerning this field this peptide is alternatively referred to as β-amyloid protein, amyloid β peptide, amyloid βA4, β protein, amyloid A4, β-peptide, and other such names.

β-amyloid peptide naturally occurs as a series of peptides which are 39 to 43 amino acids long, with the shorter, more soluble forms being present in cerebrovascular deposits and the longer forms being found primarily in senile plaques. F. Prelli, et al., Journal of Neurochemistry, 51:648-651 (1988).

Even though the full length peptide has sufficient solubility in water for the following experiments, for the purposes of convenience, a more water-soluble form of the peptide is often desired. For that reason, the following examples were performed using peptides containing just the first 40 amino acids of the β-amyloid peptide (β1-40).

It is understood by those in the art that other fragments of β-amyloid peptide, comprising amino-truncated, carboxy-truncated, or internal deletions, or any combination of these, may be employed in this invention so long as that peptide fragment demonstrates the requisite neurotoxicity.

The most frequently employed method to ensure that β-amyloid peptide adopts a β-sheet secondary structure involves "aging" of the peptide. This process comprises incubating the peptide in water or tissue culture medium for 1-10 days. The amount of time necessary for the peptide to assume a β-sheet secondary structure depends upon the constitution and pH of the buffer in which it is stored, the temperature at which the incubation occurs, the concentration of peptide in the solution and the length of the peptide employed.

### Peptide Synthesis and Purification

The peptides employed in the instant invention can be prepared using any one of many different protocols or can be purchased from commercial sources. One synthesis protocol employs stepwise solid phase peptide synthesis using a mechanical peptide synthesizer. I. Clark-Lewis, et al., Science, 231:134-139 (1986). This procedure uses commercially available N^{α}-*t*-butoxycarbonyl-protected amino acids and phenylacetamidomethyl or *p*-methylbenzhydrylamine supporting resins. The side chain blocking groups which are commonly used include: O-benzyl groups for aspartate, serine, glutamic acid, and threonine; 2-chlorocarbobenzoxy moieties for blocking lysine; bromocarbobenzoxy groups for tyrosine; tosyl groups for blocking arginine and histidine; and acetamidomethyl for cysteine. No side-chain protection is necessary for asparagine, glutamine, or methionine.

Peptides are deprotected and cleaved from the supporting resin by reaction with liquid hydrogen fluoride (in the presence of anisole) for about 1 hour at 0°C, precipitated in diethyl ether and extracted from the resin with formic acid (about 70%, v/v). To purify and desalt the peptides, crude extracts are chromatographed on size-exclusion columns equilibrated with 70% formic acid.

The peptide content of the eluate is monitored by measurement of its absorption at 280 nm. Fractions within the appropriate molecular weight range of the peptide monomer are pooled and lyophilized. A second purification is done by size-exclusion chromatography in 1 M acetic acid.

An alternative method of purifying the peptides is by the use of reversed-phase high performance liquid chromatography (RP-HPLC). The peptides are loaded onto a nonpolar stationary phase using a solvent such as 0.1% trifluoroacetic acid in water. The peptides are then eluted from the column using a gradient of increasing polarity, such as increasing concentrations of acetonitrile.

The identity and purity of the peptide is then checked using Edman degradation. The Edman degradation may be performed on whole peptides, cyanogen bromide-cleaved peptides, or protease-generated peptides. The Edman degradation may be supplemented with amino acid analysis. Alternatively the identity and purity of the synthesized peptides can be determined using mass spectrometric techniques such as electrospray, laser desorption or fast atom bombardment.

### Circular Dichroism Spectroscopy

The circular dichroism spectra can be determined using commonly known procedures. In one such procedure, solutions of peptides are mixed at room temperature for about 18 hours. If necessary, the solution is centrifuged at about 10,000 x g for about 10 minutes to produce a clear solution so that solubilized peptide can be analyzed. The CD measurements are made using a spectropolarimeter such as the AVIV 62DS. A 1 mm quartz cell is usually used for far-ultraviolet (190-240 nm) spectra. The instrument is calibrated frequently and base lines are determined. Multiple scans of each sample are obtained and the baselines deducted. Quantitative curve-fitting is done using standard reference sets. See. e.g., N. Greenfield and G. Fasman, Biochemistry, 8:4108-4116 (1969); M. Crisma, et al., International Journal of Peptide and Protein Research, 23:411-419 (1984).

### Neurotoxicity Assay Measuring Calcium Levels

An aliquot of ED 18 cortical cells were seeded into polyethylenimine-coated tissue culture dishes for 3-5 days in vitro before treatment with a 25 µM solution of β-amyloid peptide, either freshly dissolved (predominantly random coil conformation) or aged (7 days, predominantly β-sheet conformation). This neurotoxicity assay was conducted in chemically-defined HEPES-buffered DMEM supplemented with fetal calf serum.

After a two day incubation with the β-amyloid peptide, the elevation of cytosolic calcium (Ca⁺²) concentrations after a glutamate pulse were determined using a fluorescent calcium dye. J. Wahl, et al., Journal of Neurochemistry, 53:1316 (1989). The elevation of intracellular Ca⁺² levels compromises cell integrity.

### Neurotoxicity Assay Measuring XTT

An aliquot of ED 18 cortical cells were seeded into polyethylenimine-coated tissue culture dishes for 3-5 days in vitro before treatment with a 25 µM solution of β-amyloid peptide, either freshly dissolved (predominantly random coil conformation) or aged (7 days, predominantly β-sheet conformation). This assay was conducted in chemically-defined HEPES-buffered DMEM supplemented with fetal calf serum.

These cells were incubated for 3 to 5 days in vitro before treatment with a 25 µM solution of β-amyloid peptide, either freshly dissolved (predominantly random coil conformation) or aged (7 days, predominantly β-sheet conformation). After two days of incubation, cell viability was assessed by measuring the reduction of the tetrazolium salt XTT [2,3-bis(2-methoxy-4-nitro-5-sulfophenyl)-2H-tetrazolium-5-carboxanilide inner salt] as described by N. Roehm, et al., Journal of Immunological Methods, 142:257 (1992).

### Neurotoxicity Assay Measuring LDH

Primary hippocampal cultures were prepared from E18 fetal Sprague-Dawley rat pups. The cells were plated at high density (1.5 x 10⁵/cm²) in 24-well plates coated with 0.5 mg/ml of polyethylenimine. These cultures were routinely maintained in Dulbecco's Modified Eagle's Medium (DMEM) containing 4 mM glutamine, 5 g/liter glucose, 1 mM pyruvate, 20 mM potassium chloride, 50 units/ml penicillin, and 50 µg/ml streptomycin.

The cells were incubated for 10 days in vitro before treatment with a 25 µM solution of β-amyloid peptide, either freshly dissolved or aged. After four days of incubation, cell integrity was determined by measuring the level of the enzyme lactate dehydrogenase (LDH) using a standard colorimetric endpoint assay for pyruvate. P. May, et al., Neurobiology of Aging, 13:605, 606 (1992). Control standards containing normal and elevated levels of serum LDH were run with every assay.

The results of these neurotoxicity experiments demonstrates there is a direct correlation between the degree of β-sheet structure in the β-amyloid peptide and its neurotoxicity. There is minimal neurotoxicity associated with those samples of β-amyloid peptide that have a high degree of random coil in their secondary structure.

The use, therefore, of β-amyloid peptide which has adopted a predominantly β-sheet conformation allows the development of compounds which specifically inhibit the neurotoxicity. The neurotoxicity assays described, supra, can then be supplemented by the incubation of the β-amyloid peptide with potential inhibitors of neurotoxicity. The reduction in neurotoxicity can then be observed in an efficient manner.

Another embodiment of this invention encompasses conformationally-specific antibodies and antibody fragments which bind to β-amyloid peptides in a secondary structure-specific manner. Some of these antibodies bind only those β-amyloid peptides which are predominantly in a β-sheet conformation. A second set of these antibodies bind only those β-amyloid peptides which have adopted a random coil or a-helix conformation.

The term "antibody" as used in this specification describes antibodies, fragments of antibodies (such as, but not limited, to Fab, Fab', Fab₂',and Fv fragments), and chimeric, humanized, veneered, resurfaced, or CDR-grafted antibodies capable of binding antigens of a similar nature as the parent antibody molecule from which they are derived. The instant invention also encompasses single chain polypeptide binding molecules.

The term "antibody" as used in this specification is not limited by the manner in which the antibodies are produced, whether such production is in situ or not. The term "antibody" as used in this specification encompasses those antibodies produced by recombinant DNA technology means including, but not limited, to expression in bacteria, yeast, insect cell lines, or mammalian cell lines.

The synthesis of antibodies, both monoclonal and polyclonal, in animals, especially mice, is well known in the art. See, e.g., C. Milstein, Handbook of Experimental Immunology, (Blackwell Scientific Pub., 1986); J. Coding, Monoclonal Antibodies: Principles and Practice, (Academic Press, 1983). Numerous species including rats, mice, rabbits, goats, and humans are useful as a source of immunized lymphocytes for the fusion protocols utilized to generate hybridomas. BALB/c mice are especially preferred as the source of the immune cells for production of the antibodies of this invention.

It is commonplace in the field of antibody production to screen for antibodies which show a high level of specificity for defined structure such as the β-amyloid peptide in a specific conformation, while showing markedly less specificity for the same peptide having a different secondary structure. The procedures for mass screenings of hybridomas are well known in the art. See, e.g., J. Starling, et al., Cancer Immunology Immunotherapy, 28:171 (1989).

The greatest deterrence to the administration to humans of antibodies produced in non-human sources is the risk of hyperimmunogenicity due to the presence of constant regions from the species in which these antibodies are produced. Genetically engineered antibodies which retain the epitope specificity of monoclonal antibodies are now known in the art and provide a less immunogenic molecule. Such genetically engineered antibodies are contemplated in the present invention.

Chimeric antibodies are described in U.S. Patent No. 4,816,567, which issued March 28, 1989 to S. Cabilly, et al. This reference discloses methods and vectors for the preparation of chimeric antibodies. The entire contents of U.S. Patent No. 4,816,567 are herein incorporated by reference. An alternative approach to production of genetically engineered antibodies is provided in U.S. Patent No. 4,816,397, which also issued March 28, 1989 to M. Boss, et al., the entire contents of which are herein incorporated by reference. The Boss patent teaches the simultaneous co-expression of the heavy and light chains of the antibody in the same host cell.

The approach of U.S. Patent 4,816,397 has been further refined as taught in European Patent Publication No. 0 239 400, which published September 30, 1987. The teachings of this European patent publication (Winter) are the preferred format for the genetic engineering of the reactive monoclonal antibodies of this invention. The Winter technology involves the replacement of complementarity determining regions (CDRs) of a human antibody with the CDRs of a murine monoclonal antibody thereby converting the specificity of the human antibody to the specificity of the murine antibody which was the source of the CDR regions. This "CDR grafting" technology affords a molecule containing minimal murine sequence and thus is less immunogenic.

Single chain antibody technology is yet another variety of genetically engineered antibody which is now well known in the art. See, e.g. R.E. Bird, et al., Science 242:423-426 (1988); PCT Publication No. WO 88/01649, which was published 10 March 1988. The single chain antibody technology involves joining the binding regions of heavy and light chains with a polypeptide sequence to generate a single polypeptide having the binding specificity of the antibody from which it was derived.

The aforementioned genetic engineering approaches provide the skilled artisan with numerous means to generate molecules which retain the binding characteristics of the parental antibody while affording a less immunogenic format.

Some of the antibodies of the present invention demonstrate specificity for β-amyloid peptides which are predominantly β-sheet in conformation, as predominantly is defined supra. These antibodies show little binding specificity for β-amyloid peptides which have a great deal of random coil and/or a-helix in the secondary structure.

In another embodiment of this invention are antibodies which are specific for β-amyloid peptides which have adopted a random coil or α-helix conformation. These antibodies show little binding specificity for β-amyloid peptides which have a great deal of β-sheet conformation.

These antibodies are used in diagnostics, therapeutics or in diagnostic/therapeutic combinations. By "diagnostics" as used herein is meant testing that is related to either the in vitro or in vivo diagnosis of disease states or biological status in mammals, preferably in humans. By "therapeutics" and "therapeutic/diagnostic combinations" as used herein is respectively meant the treatment or the diagnosis and treatment of disease states or biological status via the in vivo administration to mammals, preferably humans, of the antibodies of the present invention. The antibodies of the present invention are especially preferred in the diagnosis and/or treatment of Alzheimer's disease in mammals, preferably humans.

In addition to being functional as direct therapeutic and diagnostic aids, the availability of a family of antibodies which are specific for β-amyloid peptide in the β-sheet conformation enables the development of numerous assay systems for detecting agents which bind to β-amyloid peptide in this specific conformation. One such assay system comprises radiolabeling β-amyloid peptide-specific antibodies with a radionuclide such as ¹²⁵I and measuring displacement of the radiolabeled β-amyloid peptide-specific antibody from solid phase β-amyloid peptide.

Numerous other assay systems are also readily adaptable to detect agents which bind β-amyloid peptide. The aforementioned assay systems are discussed in Methods in Enzymology, (J. Langone. and H. Vunakis, eds. 1981), Vol. 73, Part B, the contents of which are herein incorporated by reference. Skilled artisans are directed to Section II of Methods in Enzymology, Vol. 73, Part B, supra, which discusses labeling of antibodies and antigens, and Section IV, which discusses immunoassay methods.

The antibodies of the present invention are useful in the diagnosis and treatment of mammals suffering from Alzheimer's disease.

In another embodiment, this invention encompasses pharmaceutical formulations for parenteral administration which contain, as the active ingredient, the antibodies described, supra. Such formulations are prepared by methods commonly used in pharmaceutical chemistry.

Products for parenteral administration are often formulated and distributed in solid, preferably freeze-dried form, for reconstitution immediately before use. Such formulations are useful compositions of the present invention. Their preparation is well understood by pharmaceutical chemists.

In general, these formulations comprise the active ingredient in combination with a mixture of inorganic salts, to confer isotonicity, as well as dispersing agents such as lactose, to allow the dried preparation to dissolve quickly upon reconstitution. Such formulations are reconstituted for use with highly purified water to a known concentration.

Alternatively, a water soluble form of the antibody can be dissolved in one of the commonly used intravenous fluids and administered by infusion. Such fluids include physiological saline, Ringer's solution or a 5% dextrose solution.

## Claims

1. A method for assaying for agents which inhibit the neurotoxicity of β-amyloid peptide which comprises:
a. causing a sample of purified β-amyloid peptide to adopt a predominantly β-sheet conformation;
b. incubating potential inhibitors of neurotoxicity with the β-amyloid peptide in β-sheet conformation;
c. measuring the neurotoxic properties of each β-amyloid peptide/potential inhibitor mixture; and
d. detecting reduction in the neurotoxicity relative to a control.

2. A method as claimed in Claim 1 wherein said β-amyloid peptide has adopted 80% of its potential β-sheet conformation.

3. A method for assaying for agents which inhibit the neurotoxicity of β-amyloid peptide which comprises:
a. causing a sample of purified β-amyloid peptide to adopt a predominantly non β-sheet conformation;
b. incubating potential inhibitors of neurotoxicity with the β-amyloid peptide in non β-sheet conformation;
c. manipulating the β-amyloid peptide in such a way that β-amyloid peptide without the potential inhibitor of neurotoxicity adopts a predominantly β-sheet conformation.
d. measuring the neurotoxic properties of each β-amyloid peptide/potential inhibitor mixture; and
e. detecting reduction in the neurotoxicity relative to a control.

4. A method as claimed in Claim 5 wherein said manipulation step is aging of the peptide.

5. A method as claimed in Claim 3 wherein said β-amyloid peptide in predominantly non β-sheet conformation has adopted less than 50% of its potential β-sheet conformation.

6. A method for assaying for agents which inhibit the neurotoxicity of β-amyloid peptide which comprises:
a. allowing a sample of purified β-amyloid peptide to adopt a predominantly β-sheet conformation;
b. securing the purified β-amyloid peptide in the predominantly β-sheet conformation to a support;
c. incubating with the β-amyloid peptide an antibody which diminishes the neurotoxicity of β-amyloid peptide which is in a predominantly β-sheet conformation;
d. incubating with the β-amyloid peptide/antibody mixture a potential inhibitor of neurotoxicity;
e. subsequently measuring the amount of unbound antibody in the supernatant;
f. determining increase in the amount of unbound antibody in the supernatant relative to a control.

7. A conformationally-specific antibody which comprises an antibody having high affinity for β-amyloid peptide only when said β-amyloid peptide is in a predominantly β-sheet conformation.

8. An antibody as claimed in Claim 7 wherein said antibody is a monoclonal antibody.

9. A method of diagnosing Alzheimer's disease in a mammal which comprises administering to a mammal an antibody as claimed in Claim 7.

10. A conformationally-specific antibody which comprises an antibody having high affinity for β-amyloid peptide only when said β-amyloid peptide is in a predominantly random coil or α-helix conformation.
